(19) Europäisches Patentamt / European Patent Office / Office européen des brevets

(11) **EP 2 560 612 B1**

(12) **EUROPEAN PATENT SPECIFICATION**

(45) Date of publication and mention
of the grant of the patent:
**03.09.2014 Bulletin 2014/36**

(21) Application number: **11722553.2**

(22) Date of filing: **20.04.2011**

(51) Int Cl.:
*A61K 9/00* *(2006.01)*   *A61K 9/16* *(2006.01)*
*A61K 9/50* *(2006.01)*

(86) International application number:
**PCT/IB2011/051736**

(87) International publication number:
**WO 2011/132167 (27.10.2011 Gazette 2011/43)**

(54) **METHOD FOR PREPARING PHARMACEUTICAL COMPOSITIONS INTENDED FOR ORAL ADMINISTRATION COMPRISING ONE OR MORE ACTIVE INGREDIENTS AND THE COMPOSITIONS COMPRISING SAME**

VERFAHREN ZUR HERSTELLUNG VON PHARMAZEUTISCHEN ZUSAMMENSETZUNGEN ZUR ORALEN VERABREICHUNG MIT EINEM ODER MEHREREN WIRKSTOFFEN UND ZUSAMMENSETZUNGEN DAMIT

PROCÉDÉ DE PRÉPARATION DE COMPOSITIONS PHARMACEUTIQUES DESTINÉES À UNE ADMINISTRATION ORALE, COMPRENANT UN OU PLUSIEURS PRINCIPES ACTIFS, ET COMPOSITIONS LES COMPRENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **21.04.2010 FR 1053034**

(43) Date of publication of application:
**27.02.2013 Bulletin 2013/09**

(73) Proprietor: **SANOFI**
**75008 Paris (FR)**

(72) Inventors:
• **LESOT, Axelle**
  **F-75013 Paris (FR)**
• **LEYDET, Damia**
  **F-75013 Paris (FR)**
• **MOUSSEL, Arnaud**
  **F-75013 Paris (FR)**

(74) Representative: **Taravella, Brigitte et al**
**Sanofi**
**Département Brevets**
**54, rue La Boétie**
**75008 Paris (FR)**

(56) References cited:
EP-A1- 1 479 383   WO-A1-02/07706
WO-A1-97/18798   WO-A2-2005/065639
FR-A1- 2 753 904   FR-A1- 2 857 263
US-A- 4 948 622   US-A- 5 296 236
US-A- 5 891 476

• FAHAM A ET AL: "HOT MELT COATING TECHNOLOGY: INFLUENCE OF COMPRITOL(R) 888 ATO AND GRANULE SIZE ON CHLOROQUINE RELEASE", DIE PHARMAZIE, GOVI VERLAG PHARMAZEUTISCHER VERLAG GMBH, ESCHBORN, DE, vol. 55, no. 6, 1 June 2000 (2000-06-01), pages 444-448, XP000932193, ISSN: 0031-7144
• JOZWIAKOWSKI M J ET AL: "CHARACTERIZATION OF A HOT-MELT FLUID BED COATING PROCESS FOR FINE GRANULES", PHARMACEUTICAL RESEARCH, KLUWER ACADEMIC PUBLISHERS, NEW YORK, NY, US LNKD- DOI:10.1023/A:1015972007342, vol. 7, no. 11, 1 November 1990 (1990-11-01), pages 1119-1126, XP002052859, ISSN: 0724-8741

**Description**

## FIELD OF THE INVENTION

The present invention relates to a method for hot melt coating of pharmaceutical active ingredients. These active ingredients are characterized by organoleptic or physicochemical properties that it is desirable to mask. Thus, the pharmaceutical active ingredients may have unacceptable organoleptic properties (taste, odour, colour, appearance), in particular their strong bitterness. These pharmaceutical active ingredients may be sensitive to moisture and/or to temperature. This method therefore allows effective masking of unsatisfactory organoleptic and/or physicochemical properties of pharmaceutical active ingredients, without however slowing down the dissolution of said active ingredient. The invention also relates to the resulting medicinal active ingredients with masked organoleptic or physicochemical properties and to the compositions comprising same.

## PRIOR ART AND TECHNICAL PROBLEM

In the context of the masking of the taste of active ingredients which have an unacceptable taste, many tests, using conventional technologies, have been carried out: granulation, coating in a fluidized air bed, etc. The excipients tested were composed of polymers: mainly cellulose derivatives (HPC, HPMC, EC) and derivatives of methacrylic acid (Eudragit range) and of polyvinyl acetate. These tests often came up against insufficient taste masking.

Moreover, more innovative technologies have also been used, such as microencapsulation by means of a supercritical $CO_2$-phase process or by means of a simple or complex coacervation method. The same masking polymers, mentioned above, are found, as are other less conventional excipients such as chitosan, alginates or gelatin. The major drawbacks encountered in these technologies are implementation and industrial feasibility.

In the same approach, spray-drying and spray-cooling processes have been tested.

The latter "spray-cooling" technology described in WO/2004/058137 consists in melting a fatty matrix and incorporating the active ingredient therein. The latter is either dissolved or dispersed in the fatty phase, composed of fatty acids, of a mixture or of esters of fatty acids, of fatty alcohol or of waxes.

The molten mixture is then conveyed, via a peristaltic pump, to a two-fluid nozzle where it is atomized into more or less fine droplets (according to the parameters applied). The droplets are finally cooled by a stream of cold air within the atomization chamber and converted into solid granules containing the active ingredient dispersed throughout the fatty matrix.

Although this spray-cooling method makes it possible to obtain pharmaceutical compositions with correct taste masking while at the same time providing dissolution of the active ingredient which is not slowed down in an acidic medium, there remains, however, an unsolved problem of physical heat-stability of the product obtained. Likewise, this method remains incompatible with active ingredients which are heat-sensitive by nature.

Furthermore, in the context of this "spray-cooling" method, the concentration of the active ingredient of the composition is limited by the viscosity of the solution or suspension to be sprayed, which greatly reduces the active ingredient content of the composition of the coated granule. It is possible to obtain only compositions with low doses of active ingredient, i.e. compositions comprising an amount of active ingredient not exceeding 30% by weight in the composition of the coated granule.

Moreover, the use of the hot melt coating method has been described in WO02/07706. That document describes the use of this technology for coating heat-sensitive active ingredients in the form of solid particles which have not been pregranulated. The amounts of coating targeted are low and do not make it possible to satisfactorily mask the taste of very unpleasant active ingredients.

Likewise, WO9718798 describes prompt-release pharmaceutical compositions suitable in particular for oral administration, comprising microcrystals or microgranules of active agents, a lipid coating obtained by means of a hot melt coating method and a vehicle comprising excipients. The concentration of fatty matrix of the coating is between 1% and 25%, preferably between 5% and 20%. That document specifies that, in order to mask the unpleasant taste of an active ingredient while at the same time allowing satisfactory release of the active agent, it is necessary to work with sufficiently low levels of fatty matrix. This condition is necessary so as not to modify the dimensions of the coating layer, which would then influence the release of the active ingredient. However, the amounts of lipid coating proposed are low and do not make it possible to satisfactorily mask the taste of very unpleasant active ingredients.

As a result, the use of the hot melt coating method described in these documents is not suitable for obtaining sufficiently effective masking of the taste and of the odour of an active ingredient characterized by a strong bitterness and a strong odour, in particular masking sufficient to allow ingestion of the medicament by a child.

The applicant has now found a novel improved method which makes it possible to solve these drawbacks.

## SUMMARY OF THE INVENTION

The objective of the invention is to propose a novel method which solves at least the drawbacks mentioned above.

The problem solved by this method of production which comprises formulation using fatty matrices, is that of the masking of the taste of certain active ingredients while at the same time obtaining a relatively rapid release of the active ingredient in vitro (for example a minimum release of 70% of active ingredient in 60 minutes).

Furthermore, the maximum concentration of active ingredient that can be used in this method is not limited; the resulting product can contain a high dose, i.e. it is possible to obtain compositions that can comprise an amount of active ingredient exceeding 30% by weight in the composition of the coated granule. This makes it possible to notably reduce the doses of products to be administered to the patient, in particular to young children or to the elderly.

According to an additional advantage, this method makes it possible to obtain a high dosage of the active ingredient even when the latter is heat-sensitive. Furthermore, it is observed that the final products obtained exhibit satsifactory physical stability, in particular under storage conditions subjected to heat (at 30°C/65% RH).

Moreover and surprisingly, this method makes it possible to provide certain sensitive active ingredients with protection against moisture. This is because the presence of this lipid coating appears to make it possible to establish a barrier against moisture.

According to another additional advantage, this method makes it possible, by virtue of the quality of the coating, to inhibit the anaesthetic and unpleasant effect of certain active molecules generally felt in the oral cavity.

The invention therefore proposes a method for preparing a composition of medicinal active ingredient comprising (a) a granular centre consisting of grains of active ingredient, agglomerated in the presence of binder, and (b) a layer of coating of said granular centre consisting of fatty matrix, in which composition:

- the active ingredient represents a minimum of 10%, preferably 20% or else 30% by weight, relative to the weight of the composition of the coated granule; the active ingredient represents a maximum of 48%, preferably a maximum of 40%, the active ingredient preferably represents from 20% to less than 40%, relative to the weight of the composition of the coated granule, more preferentially the active ingredient represents from 30% to 40%, relative to the weight of the composition of the coated granule;
- the fatty matrix represents more than 50% and up to 85% by weight of the composition of the coated granule, preferably from 51% to 65%, the fatty matrix optionally comprising an adjuvant, preferably chosen from hydrophilic agents, surfactants or mixtures thereof, and the latter representing less than 10%, preferably from 1% to 3% by weight, relative to the weight of the composition of the coated granule;
- the binder, preferably a hydrophilic agent chosen from hydrophilic polymers or hot-melt agents, represents from 0.2% to 18% by weight, relative to the weight of the composition of the coated granule, preferably the binder represents less than 18% by weight for a hot-melt agent or else preferably the binder represents less than 10% by weight for a hydrophilic polymer;
- the diluent, if necessary, as filler, represents a content of 0 to 39%, relative to the weight of the composition of the coated granule;
- the lubricant, if necessary, as flow agent, represents a content of 0 to 1.8%, relative to the weight of the composition of the coated granule;

the method comprising the steps of:

E1) preparing the granular centre by spraying an aqueous solution comprising the binder or by spraying the binder in the molten state onto the active ingredient alone or as a mixture with a diluent and/or a lubricant;
E2) coating by spraying, onto the granules, said fatty matrix pre-melted in a melting kettle at a temperature approximately 10 to 20°C above its melting point;
E3) cooling the composition obtained.

According to one preferred embodiment, the method makes it possible to prepare a composition in which:

- the active ingredient represents a minimum of 10% and a maximum of 48%, preferably from 20% to less than 40% by weight of the composition of the coated granule, and
- the fatty matrix comprising an adjuvant represents more than 50% and up to 85% by weight of the composition of the coated granule.

According to another preferred embodiment, the method makes it possible to prepare a composition in which:

- the active ingredient represents from 30% to 40%, relative to the weight of the composition of the coated granule, and

- the fatty matrix represents from 51% up to 65% by weight of the composition of the coated granule.

Preferably, the granular centre of active ingredient is coated only with a single layer of coating.

Preferably, the size of the coated granules obtained at the end of step E3) is less than 500 $\mu$m, preferably less than 355 $\mu$m, preferably ranging from 100 to 300 $\mu$m.

Preferably, the particle size of the final product obtained at the end of step E3) is distributed according to the following range:

- less than 15% by weight of the coated granules are greater than 500 $\mu$m;
- more than 80% by weight, preferably more than 90% by weight, of the coated granules are between 355 and 90 $\mu$m; and
- less than 20% by weight, preferably less than 5% by weight, of the coated granules are less than 90 $\mu$m.

Preferably, the aqueous solution used in step E1 comprises, as binder, a hydrophilic polymer preferably chosen from the group of cellulosic derivatives (hydroxypropylcellulose), povidone (polyvinylpyrrolidone), sucrose, gums, starches, gelatin and macrogols (polyethylene glycols), which represents approximately from 15% to 45%, preferably 20% to 40% by weight of said aqueous solution.

Preferably, the binder used in step E1 for the granulation is a hot-melt agent chosen from macrogols (PEGs), sucroesters or else poloxamers, and represents approximately from 0.2% to 20%, preferably from 1% to 15% by weight, relative to the amount of active ingredient to be granulated in step E1).

According to one particular embodiment, the aqueous solution used in step E1 is sprayed onto the active ingredient mixed with a diluent (filler). The diluents used in granulation for increasing the load to be granulated are preferably chosen from polyols, celluloses, sugars, lactoses, starches, kaolin, calcium phosphates, calcium carbonates or magnesium carbonates, or derivatives thereof. This diluent represents approximately from 0 to 39%, preferably 5% to 15% by weight of the composition of the coated granule. Said diluent may optionally act as a permeabilizing agent thus facilitating dissolution of the active ingredient.

Preferably, the fatty matrix consists of saturated fatty acids with long C14 to C22, preferably C16 to C18, carbon-based chains, pure or as mixtures, and/or their corresponding fatty alcohols.

Preferably, the fatty matrix consists of stearic acid, palmitic acid, myristic acid, pure or as mixtures, and/or their corresponding fatty alcohols. More preferentially, the fatty matrix consists only of stearic acid, which is a C18 saturated fatty acid.

Preferably, the adjuvant as a mixture with the fatty matrix is chosen from the group of surfactants (phospholipid, polysorbate, lauryl sulphate), hydrophilic excipients such as sucrose, polyols, cellulose, lactose, silica, dicalcium phosphate, carbonates, starch, macrogols and agents which are soluble at acidic pH (methacrylic derivatives), pure or as mixtures. Preferably, the adjuvant used is a glycerolipid, in particular a phospholipid. More preferentially, this phospholipid is a lecithin (phosphatidylcholine), preferably soybean lecithin.

Said fatty matrix coating the granular centre preferably consists of stearic acid and the adjuvant as a mixture with the fatty matrix is soybean lecithin.

Preferably, the percentage by weight of the adjuvant added to the fat in step E2 is less than 10% by weight, preferably less than 5% by weight, preferably ranging from 1% to 3% by weight, relative to the weight of the composition of the coated granule.

Preferably, the percentage by weight of the binder constituting the coating of the granule obtained in step E1 represents from 1% to 5% by weight, relative to the weight of the composition of the coated granule, for a hydrophilic polymer and from 0.2% to 18% for a hot-melt agent.

Preferably, the active ingredient is chosen from the group consisting of antibiotics such as cephalosporins and macrolides, advantageously chosen from: pristinamycin, cefpodoxime, roxithromycin, spiramycin, rovamycin and levofloxacin, or the group consisting of corticoids such as prednisolone or methylprednisolone, or else the group consisting of NSAIDs such as tiaprofenic acid, ketoprofen, ketoprofen lysinate or ibuprofen, or the group consisting of analgesics such as paracetamol, or else the group consisting of zopiclone, riluzole, zolpidem, clobazam, thiocolchicoside, drotaverine hydrochloride or base, amodiaquine hydrochloride, diltiazem, levocetirizin, mizolastine, dronedarone, celivarone, dramamine, ramipril, irbesartan, clopidogrel, leflunomide, nicorandil, lysine acetyl salicylate, magnesium citrate, levothyroxine, sodium valproate, rifampicin, artesunate and omeprazole.

Preferably, the method is followed by a step E4 of formulating the coated granules obtained in step E3 with excipients such as diluents, fillers, viscosity modifiers, disintegrating agents, effervescent agents, colourants, sweeteners, salivating agents, flavourings, buffers and sequestering agents, flow agents and lubricants for producing an oral form in the form of granules for sachets, granules for oral suspension, or granules for conventional tablets or for orodispersible tablets.

According to another subject, the invention relates to a composition of medicinal active ingredient comprising (a) a granular centre consisting of grains of active ingredient agglomerated in the presence of binder and, optionally, of diluent or of lubricant, and (b) a layer of coating of said granular centre consisting of a fatty matrix, in which composition:

- the active ingredient represents a minimum of 10%, preferably 20% or else 30% by weight, relative to the weight of the composition of the coated granule; the active ingredient represents a maximum of 48%, preferably a maximum of 40%, the active ingredient preferably represents from 20% to less than 40%, relative to the weight of the composition of the coated granule, more preferentially the active ingredient represents from 30% to 40%, relative to the weight of the composition of the coated granule;
- the fatty matrix represents more than 50% and up to 85% by weight of the composition of the coated granule, preferably from 51% to 65%, the fatty matrix optionally comprising an adjuvant, preferably chosen from hydrophilic agents, surfactants or mixtures thereof, and the latter representing less than 10%, preferably from 1% to 3% by weight, relative to the weight of the composition of the coated granule;
- the binder, preferably a hydrophilic agent chosen from hydrophilic polymers or hot-melt agents, represents from 0.2% to 18% by weight, relative to the weight of the composition of the coated granule, preferably the binder represents less than 18% by weight for a hot-melt agent or else preferably the binder represents less than 10% by weight for a hydrophilic polymer;
- the diluent, if necessary, as filler, represents a content of 0 to 39%, relative to the weight of the composition of the coated granule;
- the lubricant, if necessary, as flow agent, represents a content of 0 to 1.8%, relative to the weight of the composition of the coated granule.

Preferably, in the composition of medicinal active ingredient according to the invention, said fatty matrix consists of saturated fatty acids with long C14 to C22, preferably C16 to C18, carbon-based chains, pure or as mixtures, and/or their corresponding fatty alcohols, and the binder is chosen from hydrophilic polymers. More preferentially, the fatty matrix consists only of stearic acid, which is a C18 saturated fatty acid.

Preferably, said fatty matrix comprises an adjuvant chosen from the group of surfactants (phospholipid, polysorbate, lauryl sulphate), hydrophilic excipients such as sucrose, polyols, cellulose, lactose, silica, dicalcium phosphate, carbonates, starch, macrogols and agents which are soluble at acidic pH (methacrylic derivatives), pure or as mixtures, preferably phospholipids. Preferably, the adjuvant used is a glycerolipid, in particular a phospholipid. More preferentially, this phospholipid is a lecithin (phosphatidylcholine), preferably soybean lecithin.

Preferably, said fatty matrix coating the granular centre consists of stearic acid and the adjuvant as a mixture with the fatty matrix is soybean lecithin.

Preferably, the adjuvant is a surfactant which represents less than 10% by weight, preferably less than 5% by weight, preferably from 1% to 3% by weight, relative to the weight of the composition of the coated granule.

Preferably, the active ingredient is chosen from the group consisting of antibiotics such as cephalosporins and macrolides, advantageously chosen from: pristinamycin, cefpodoxime, roxithromycin, spiramycin, rovamycin and levofloxacin, or the group consisting of corticoids such as prednisolone or methylprednisolone, or else the group consisting of NSAIDs such as tiaprofenic acid, ketoprofen, ketoprofen lysinate or ibuprofen, or the group consisting of analgesics such as paracetamol, or else the group consisting of zopiclone, riluzole, zolpidem, clobazam, thiocolchicoside, drotaverine hydrochloride or base, amodiaquine hydrochloride, diltiazem, levocetirizin, mizolastine, dronedarone, celivarone, dramamine, ramipril, irbesartan, clopidogrel, leflunomide, nicorandil, lysine acetyl salicylate, magnesium citrate, levothyroxine, sodium valproate, rifampicin, artesunate and omeprazole.

Preferably, the amount of active ingredient represents more than 10% by weight and ranges up to 48% by weight, relative to the weight of the composition of the coated granule.

Preferably, the amount of active ingredient represents more than 10% by weight of the composition of the coated granule and the fatty matrix comprises an adjuvant.

Preferably, the amount of active ingredient represents from 20% to less than 40% by weight of the composition of the coated granule.

Preferably, the composition of the active ingredient according to the invention is obtained by means of the method as described above.

According to another subject, the invention relates to a pharmaceutical composition comprising the composition of medicinal active ingredient as described above or else which can be prepared according to the method described above.

According to another subject, the invention relates to a sachet for an oral suspension comprising the composition of active ingredient as defined above in the presence of an excipient chosen from diluents, viscosity modifiers, disintegrating agents, sequestering agents, buffers, preservatives, lubricants, wetting agents, effervescent agents, colourants, sweeteners, salivating agents or flavourings.

According to another subject, the invention relates to tablets to be chewed, swallowed or sucked, or orodispersible or water-dispersible tablets with a masked taste, comprising the composition of active ingredient as defined above in the presence of an excipient chosen from diluents, binders, lubricants, salivating agents, anaesthetic agents, wetting agents, preservatives, flow agents, disintegrating agents, colourants, sweeteners or flavourings.

According to another subject, the invention relates to a powder to be swallowed, with masked taste, comprising the composition of active ingredient as defined above in the presence of an excipient chosen from diluents, salivating agents, effervescent agents, flow agents, preservatives, colourants, sweeteners or flavourings.

According to another subject, the invention relates to the use of a composition of medicinal active ingredient as defined above or prepared according to the method described above, for preparing pharmaceutical compositions in the form of sachets for an oral suspension, powders to be swallowed, tablets to be chewed, tablets to be swallowed, tablets to be sucked, orodispersible tablets or water-dispersible tablets, and having a masked taste.

## DETAILED DESCRIPTION OF EMBODIMENTS OF THE INVENTION

### Method for preparing the composition of medicinal active ingredient

### 1) Granulation step

In a first step of the method, an aqueous wetting solution comprising a binder of hydrophilic polymer type is sprayed onto the active ingredient in the crude state or onto an active ingredient/diluent (filler) mixture or onto an active ingredient/lubricant mixture or an active ingredient/diluent/lubricant mixture.

Preferably, the aqueous solution contains approximately 10% to 45% by weight, preferably 15% to 40% by weight, of binder of hydrophilic polymer type in the aqueous solution.

The parameters used for this granulation step are suitable for the properties of the active ingredient and for the equipment used.

The aqueous wetting solution used can be replaced, when moisture-sensitive active ingredients are used, with a hot-melt binder (of macrogol type).

This step makes it possible to obtain an active ingredient which is finely granulated by homogenization and recentring of the particle size distribution of the active ingredient, with the aim of improving the quality of the second, coating step.

According to one variant, it is also possible to carry out a calibration of the granular active ingredient obtained in the step above, in order to select the granules having a diameter of less than 500 $\mu$m.

### 2) Hot-melt coating step

In a second step, the active ingredient in the granular state is preferably fluidized in a fluidized air bed.

The fatty matrix is brought to melting, with stirring, in a melting kettle at a temperature of approximately 10 to 20°C above its melting point.

The melting points of the fatty substances used are in the region of from 50 to 80°C, preferentially from 55 to 65°C or else preferably around 60°C. This melting range was preferably chosen, firstly, for reasons of physical stability of the composition thus formulated and, secondly, in order to have, in the end, as prompt a release of active ingredient as possible.

According to one preferred embodiment, an adjuvant (surfactant or hydrophilic excipient) is added to the melted fatty matrix with, optionally, a preservative. This adjuvant makes it possible to promote the obtaining of a prompt release profile of the composition.

The molten mixture is then sprayed onto the active ingredient in order to produce a coating by the "hot-melt coating" method. The parameters applied during the coating method are summarized below.

The coating method can be carried out in a fluidized air bed apparatus. The nozzles are fed, on the one hand, with the molten mixture which circulates via a pump in an entirely thermally-insulated and completed circuit, and secondly, with hot compressed air fed via an air heater.

The main operating parameters applied, which are suitable for the equipment used and the batch size used, are the following:

- T° of the fatty substance melted: from 70 to 95°C, according to the properties and characteristics of the fatty matrix;
- T° compressed air for spraying: from 80 to 120°C;
- Compressed air pressure: from 0.5 to 1.5 bar;
- T° of the feed circuits: between 80 and 100°C;
- Liquid flow rate: from 5 to 30 g/min;
- Air flow rate: this is usually adjusted according to the size of the equipment. For an apparatus with a working capacity of 3 kg, an air flow rate of 50 to 160 m³/h is generally used, according to the equipment filling load;

- T° air inlet: from 25 to 50°C, according to the properties of the fatty matrix used.

At the end of these steps and after cooling, a composition of medicinal active ingredient comprising a granular centre consisting of grains of active ingredient agglomerated in the presence of binder, and a layer of coating of said granular centre consisting of fatty matrix, is obtained.

Preferably, the granular centre of active ingredient is coated only with a single layer of coating.

**Description of the composition of medicinal active ingredient,**

**1. Active ingredient**

The invention relates to any type of active ingredient in the form of solid particles intended to be coated in order to mask their unsatisfactory organoleptic or physicochemical properties.

The active ingredient used in the invention in particular has unpleasant organoleptic properties (taste, odour, appearance), an unacceptable taste and/or an unacceptable odour, or else it can have an anaesthetic, hot, irritant or astringent effect when it passes through the oral cavity. In addition, the active ingredient used in the invention may also be heat-sensitive or else moisture-unstable.

It is preferably chosen from the group consisting of antibiotics such as cephalosporins and macrolides, advantageously chosen from: pristinamycin, cefpodoxime, roxithromycin, spiramycin, rovamycin and levofloxacin, or the group consisting of corticoids such as prednisolone or methylprednisolone, or else the group consisting of NSAIDs such as tiaprofenic acid, ketoprofen, ketoprofen lysinate or ibuprofen, or the group consisting of analgesics such as paracetamol, or else the group consisting of zopiclone, riluzole, zolpidem, clobazam, thiocolchicoside, drotaverine hydrochloride or base, amodiaquine hydrochloride, diltiazem, levocetirizin, mizolastine, dronedarone, celivarone, dramamine, ramipril, irbesartan and clopidogrel.

Among the moisture-sensitive active ingredients, mention may be made of: leflunomide, nicorandil, lysine acetyl salicylate, ramipril, magnesium citrate, levothyroxine, sodium valproate, rifampicin, artesunate, clopidogrel and omeprazole.

Amisulpride is excluded from the list of active ingredients used in the invention.

According to one preferred embodiment, the active ingredient used in the invention is chosen from the group consisting of drotaverine hydrochloride or base, clobazam, paracetamol, riluzole, ketoprofen, ketoprofen lysinate, clopidogrel, irbesartan, zopiclone, zolpidem and pristinamycin.

**2. Active ingredient granulating agents**

The granulating agents used in the first step of the method are binders preferably chosen from hydrophilic agents. They are used in a proportion of from 0.2% to 18% by weight, relative to the weight of the composition of the coated granule.

These hydrophilic agents are particularly chosen from the group of cellulosic derivatives (hydroxypropylcellulose), povidone (polyvinylpyrrolidone), sucrose, gums, starches, gelatin, macrogols, sucroesters and poloxamers.

According to one embodiment, use is in particular made of hydrophilic polymers, preferably PEG or PVP in an aqueous solution in a proportion of from approximately 10% to 45% by weight in the aqueous solution.

Preferably, when the binder is a hydrophilic polymer, the percentage by weight of the binder is less than 10% by weight, preferably less than 5% by weight, preferably ranging from 1% to 5% by weight, relative to the weight of the composition of the coated granule.

According to another embodiment, use is made of hydrophilic agents chosen from hot-melt agents (melting point < 85°C) such as macrogols (PEG), sucroesters or else poloxamers, in particular when moisture-sensitive active ingredients are used. The concentrations of hot-melt binders used are about from 0.2% to 20%, preferably from 1% to 15% by weight, relative to the amount of active ingredient to be granulated.

Preferably, when the binder is a hot-melt agent, the percentage by weight of the binder is less than 18% by weight, preferably less than 13.5% by weight, relative to the weight of the composition of the coated granule, and preferably from 0.2% to 13.5% by weight, relative to the weight of the composition of the coated granule.

The diluents used in granulation in order to increase the load to be granulated or else in order to facilitate the dissolution of the active ingredient are preferably chosen from polyols, celluloses, sugars, lactoses, starches, kaolin, calcium phosphates, calcium carbonates or magnesium carbonates, or mixtures thereof. These diluents are present in a content of between 0 and 80% of the weight of the granule, which represents 0 to 39% by weight of the weight of the composition of the coated granule.

The lubricants used in granulation in order to improve the fluidization are preferably chosen from silicas and talc. These lubricants are present in a content of between 0 and 2% of the weight of the granule, which represents 0 to 1.8% by weight of the weight of the composition of the coated granule.

### 3. Characteristics of the granular active ingredient

The granule comprising the active ingredient and the binder, obtained at the end of the first granulation step, has a particle size of less than 500 $\mu$m, preferably on average less than 200 $\mu$m.

### 4. Fatty matrix

The fatty matrices are chosen from the group of saturated fatty acids with long C14 to C18, preferably C16 to C18, carbon-based chains, pure or as mixtures, and/or their corresponding fatty alcohols and/or the corresponding esters of fatty acids and alcohols. Preferably, the fatty matrix consists of stearic acid, palmitic acid, myristic acid, pure or as mixtures, and/or their corresponding fatty alcohols. More preferentially, the fatty matrix consists only of stearic acid, which is a C18 saturated fatty acid.

Preferably, the fatty matrix represents more than 50% by weight of the composition and up to 85% by weight of the composition; more preferentially from 51% to 65% by weight of the composition of the coated granule.

According to one preferred embodiment, use is made, as fatty matrix, of stearic acid in a proportion of more then 50% by weight, relative to the weight of the composition of the coated granule, preferably from 51% to 65% in order to improve the masking of the taste of very unpleasant molecules.

### 5. Fatty matrix adjuvants

The adjuvants are chosen from the group of surfactants such as phospholipids, polysorbate or lauryl sulphate, hydrophilic excipients such as sucrose, polyols, cellulose, lactose, silica, dicalcium phosphate, starch, povidones or macrogols, and agents which are soluble at acidic pH, such as methacrylic derivatives; preferably, phospholipids are used. Preferably, the adjuvant used is a glycerolipid, in particular a phospholipid. More preferentially, this phospholipid is a lecithin (phosphatidylcholine), preferably soybean lecithin.

Preferably, the percentage by weight of the adjuvant added to the fat in the coating step is less than 10% by weight, preferably less than 5% by weight, preferably ranging from 0.5% to 3.5% or else from 1% to 3% by weight, relative to the weight of the composition of the coated granule.

According to one preferred embodiment, phospholipids are used as adjuvant, preferentially soybean lecithin, in a proportion of from 1% to 3% by weight, relative to the weight of the composition of the coated granule, in order to improve the dissolution profile.

### <u>Characteristics of the composition of medicinal active ingredient:</u>

### Particle size

The particle sizes of the products resulting from this method are about a few hundred microns (according to the parameters applied and the type of equipment used).

The particle size distribution of the product obtained is distributed according to the following range:

o less than 15% by weight of the coated granules are greater than 500 $\mu$m;
o more than 80% by weight, preferably more than 90% by weight, of the coated granules are between 355 and 90 $\mu$m; and
o less than 20% by weight, preferably less than 5% by weight, of the coated granules are less than 90 $\mu$m.

### Preferred amounts and ratio

The composition according to the invention consists of medicinal active ingredient comprising (a) a granular centre consisting of grains of active ingredient agglomerated in the presence of binder, and (b) a layer of coating of said granular centre consisting of fatty matrix.

In this composition, the preferred amounts and ratios of the various combinations of ingredients are represented below:

- the active ingredient represents a minimum of 30% by weight relative to the weight of the composition of the coated granule; the active ingredient represents a maximum of 48%, preferably a maximum of 40%, the active ingredient preferably represents from 30% to 40%, relative to the weight of the composition of the coated granule;

- the fatty matrix represents more than 50% and up to 85% by weight of the composition of the coated granule, preferably from 51% to 65%, the fatty matrix optionally comprising an adjuvant, preferably chosen from hydrophilic agents, surfactants or mixtures thereof, and the latter representing less than 10% by weight of the composition, preferably from 1% to 3% by weight, relative to the weight of the composition of the coated granule;
- the adjuvant, when it is present in the fatty matrix, represents less than 10% by weight of the composition, preferably from 1% to 3% by weight, relative to the weight of the composition of the coated granule;
- the binder, preferably a hydrophilic polymer or a hot-melt agent, represents less than 18% (for a hot-melt agent) or else less than 10% (for a polymer) by weight, relative to the weight of the composition of the coated granule;
- the diluent, if necessary, as filler, represents a content of from 0 to 39% by weight, relative to the weight of the composition of the coated granule;
- the lubricant, if necessary as flow agent, represents a content of from 0 to 1.8% by weight, relative to the weight of the composition of the coated granule.

**Properties**

The formulation of the coated granule according to the invention allows effective masking of the taste and of the odours of active ingredients with unpleasant organoleptic properties. This is because the coating of the active ingredient allows the creation of a barrier around said active ingredient so as to mask its taste, its odour and, potentially, its colour.

In parallel, products having a relatively prompt release of the active ingredient in vitro are obtained (for example a minimum release of 70% in 60 min).

Moreover, by virtue of the effective taste masking, it is possible to obtain products with a high dose of active ingredient, for instance containing more than 30% of active ingredient, which makes it possible to notably reduce the doses of products to be administered to the patient, in particular to young children or to the elderly.

According to another advantage, this method makes it possible to combine a high dosage of the active ingredient with heat-sensitive or moisture-sensitive active ingredients.

In addition, it is observed that the physical appearance of the final products obtained remains stable during heat-stability tests (30°C/65%RH).

Furthermore, it is observed that the composition of the coated granule exhibits good stability with respect to moisture.

According to another additional advantage, this method makes it possible, by virtue of the quality of its coating, to inhibit the anaesthetic, irritant, astringent and unpleasant effect in the mouth of certain active molecules.

Evaluation of the taste masking:

The taste masking produced by this coating can be evaluated:

- qualitatively, by means of tasting tests carried out by volunteers;
- quantitatively, using analytical methods such as the "glass of water test" in which the amount of active ingredient released at a given time is assayed and a comparison is made with a given bitterness threshold.

The method of the glass of water test, making it possible to replace the tasting test, is described below for the example of pristinamycin:

| Medium | demineralized water + 0.2% SDS |
| --- | --- |
| Volume | 50 ml |
| Concentration of active ingredient | 10000 mg/l |
| Stirring | 50 rpm |
| Temperature | ambient |
| Samples | at 5, 10 and 15 minutes |
| Filtration | double filtration through 0.45 $\mu$m + 0.22 $\mu$m Millipore |
| Detection | range 240-270 nm |

(continued)

| Cuvette | 0.1 cm |
| Measurement | without dilution |

*Calculation of the concentration in the glass of water:*

The concentration of pristinamycin dissolved at time "t" ($C_{granule}$) is calculated according to the measurement of absorbance at 252 nm in the glass of water $A_{granule}$ and of the pristinamycin control ($A_{pyo\ control}$) at the concentration $C_{control}$:

$$C_{granule}\ (mg/L) = (A_{granule} \times C_{control}) / A_{pyo\ control}$$

The bitterness threshold for pristinamycin is set at 400 mg/L released in 15 minutes.

In vitro dissolution/release:

The dissolution profiles for the composition obtained at the end of the method are determined according to the method of the European Pharmacopoeia 2.9.3.

Physical stability

The appearance of the product after 3 months at 30°C and 65%RH is examined.
When the product is nonagglomerated, the result is denoted satisfactory (++) or when the product is agglomerated, the result is denoted unsatisfactory (- -).

Moisture-stability

The appearance of the granule coated in accordance with the method according to the invention is examined when said granule is placed under accelerated degradation conditions, i.e. in a controlled chamber at 40°C and 75% relative humidity (RH).
The stability of the active agent is measured by assaying the degradation impurities produced from said active agent.
The behaviour with respect to moisture, of the product resulting from the method of the invention, is compared at various coating contents.

Anaesthetic effect test:

A taste test is carried out by volunteers. The test tablets are broken in half and then placed in the mouth. The anaesthetic effect in the oral cavity is assessed after several minutes following administration of the half-tablet.
When the anaesthetic effect is no longer felt, the result is denoted satisfactory (++) or when the anaesthetic effect persists, the result is denoted unsatisfactory (- -).
The molecules which have an anaesthetic effect and which are exemplified in the compositions tested are drotaverine and riluzole.

Formulation of the external phase

The granule coated with fatty matrix, obtained according to the method of the invention, may subsequently be integrated into an external formulation for the manufacture of an oral form, such as granules for a sachet, granules for suspension, tablets to be chewed, tablets to be sucked, tablets to be swallowed or else orodispersible tablets or granules.
In the case of the formulation of sachets, the formulation of the external phase can be enriched with surfactants or wetting agents in order to improve the resuspension of the granules obtained in an aqueous medium.
Other excipients, such as fillers or diluents, colourants, sweeteners, viscosity modifiers or gelling agents, adsorbents, buffers or flavourings may also be added to the formulation for the purpose of improving the final appearance of the product.

## Examples

The following examples illustrate the present invention without, however, limiting the scope thereof.

### A. Preparation of the granular medicinal active ingredients coated with a fatty matrix

Various active ingredients characterized by their strong unpleasant odour and/or their very pronounced bitterness or else having an anaesthetic, hot, irritant or astringent effect were used in the tests.

### 1. Description of the hot melt method for obtaining the compositions according to the invention

Three kilos of pristinamycin in the crude state are fluidized in a fluidized air bed.

Over the course of a few minutes of fluidization at an average flow rate of 90 m$^3$/h, spraying is initiated at an average flow rate of 30 g/min.

The granulation solution is composed of 1.4 kg of water in which 600 g of PEG 6000 fine powder are dispersed, i.e. a concentration of 30% of binder.

The inlet temperature is set at 75°C for a product and outlet temperature equal to 40°C. The spraying lasts approximately 1 hour. The grain obtained is finely granulated and has a particle size predominantly less than 200 μm.

In the case of moisture-sensitive active ingredients, the granulation can be carried out directly with PEG 6000 alone in the molten state.

The fatty matrix consisting of stearic acid is melted at 80°C. After homogenization of the fatty substance, the phospholipid adjuvant, in an amount of from 2% to 5% according to the formulations, is added to the molten fatty matrix until complete homogenization of the mass is obtained.

Stirring is maintained throughout the duration of spraying.

A few hundred grams of granules are fluidized in the fluidized air bed with an air flow rate of 70 to 90 m$^3$/h (the latter is adjusted according to the progression of the step, i.e. according to the load and the density gradually acquired by the grain).

The inlet air temperature during the coating is set between 30° and 40°C. The molten mixture is then sprayed at a flow rate of 15 g/min on average.

Once set, this flow rate remains constant throughout the duration of the spraying. The spray pressures used are themselves also kept constant and are between 0.5 and 0.9 bar. The air used is heated to a target temperature of 90°C.

The spraying time varies according to the formula. Once the spraying is complete, the inlet air temperature is cut and the granule is thus cooled before being discharged.

### 2. Influence of the fatty matrix and of the adjuvant in a composition of active agent according to the invention on the particle size, the masking of bitterness, the dissolution, the physical stability and the anaesthetic effect of the active ingredient

Tables I and II represent all the formulations tested from C1 to C20.

| Table I | | | | | | | | | | | | |
|---|---|---|---|---|---|---|---|---|---|---|---|---|
| Ingredients | % by weight | C1 | C2 | C3 | C4 | C5 | C6 | C7 | C8 | C9 | C10 | C11 |
| Active ingredient | pristinamycin | 32.5 | 34.6 | 32.8 | 29 | 25.1 | 25.2 | 24.7 | 25 | 18.3 | 20.8 | 23.4 |
| Lubricant | aerosil 200 | 0.3 | 0.4 | 0.3 | 0.3 | 0.3 | 0.3 | 0.3 | | | | |
| Binder | PEG 600 | 6.6 | 7 | 6.6 | 5.9 | 5.1 | 5.1 | 5 | 5 | 3.7 | 4.2 | 4.7 |
| Fatty matrix | stearic acid | | 60 | | 64.8 | 64.5 | 64.4 | 65 | 65 | 75 | 73 | 70 |
| | stearyl alcohol | | | 60.3 | | | | | | | | |
| | palmitic acid | 60.6 | | | | | | | | | | |
| Adjuvant | phospholipid | | | | | 5 | | | 2 | 3 | 2 | 2 |
| | polysorbate | | | | | | | 5 | | | | |
| | PEG 6000 | | | | | | 5 | | | | | |

(continued)

| Results | | | | | | | | | | | |
|---|---|---|---|---|---|---|---|---|---|---|---|
| Particle size profile | ++ | ++ | ++ | ++ | ++ | -- | ++ | ++ | ++ | ++ | ++ |
| Bitterness masking | ++ | ++ | ++ | ++ | ++ | ++ | -- | ++ | ++ | ++ | ++ |
| Dissolution profile T 0 | -- | ++ | ++ | -- | ++ | -- | -- | ++ | ++ | ++ | ++ |
| Physical stability T 3 months 40°C/75%RH | ++ | ++ | ++ | / | / | / | / | ++ | / | / | / |
| Dissolution profile T 3 months 30°C/65%RH | -- | ++ | -- | / | / | / | / | / | / | / | / |

| Table II | | | | | | | | | |
|---|---|---|---|---|---|---|---|---|---|
| Ingredients | % by weight | C12 | C13 | C14 | C15 | C16 | C17 | C18 | C19 | C20 |
| Active ingredient | drotaverine | 30 | 29.5 | 28 | | | | | | |
| | clobazam | | | | 18 | 14 | 16 | | | |
| | riluzole | | | | | | | 43 | | |
| | clopidogrel | | | | | | | | | 32.3 |
| | ketoprofen | | | | | | | | 35 | |
| Diluent | mannitol | | | | 25 | 19.5 | 22.3 | | | |
| Lubricant | colloidal silica | | | | | | | 0.9 | | |
| Binder | PVP | 4 | 4.5 | 4 | 2 | 1.5 | 1.7 | 5.1 | 1.85 | |
| | PEG | | | | | | | | | 5.7 |
| Fatty matrix | stearic acid | 65 | 65 | 65 | 55 | 65 | 59 | 51 | 60 | 60 |
| Adjuvant | phospholipid | | 1 | 3 | | | 1 | | 2 | 2 |
| Results | | | | | | | | | | |
| Particle size profile | | ++ | ++ | ++ | ++ | ++ | ++ | ++ | ++ | |
| Taste masking | | ++ | ++ | ++ | ++ | ++ | + | ++ | ++ | |
| Anaesthetic effect inhibition | | ++ | ++ | ++ | N | N | N | ++ | N | N |
| Dissolution profile T0 | | - | | ++ | / | ++ | / | ++ | ++ | |
| ++: Satisfactory result      -: Unsatisfactory result      /: Absence of result    N: Not concerned | | | | | | | | | | |

## Conclusions

**1) Presence of the adjuvant:**

It may be noted that the addition of an adjuvant to the formulation in the fatty matrix, of phospholipid type added in an amount of a few percent, 2 to 5%, makes it possible to accelerate dissolution.

**2) Choice of fatty substance:**

It may be noted that, according to the type of fatty substance, for the same masking quality, the dissolution profiles and the physical heat-stability are not equivalent (cf. Examples C1, C2, C3). The best compromise is obtained with stearic acid, which is a C18 saturated fatty acid.

**3) Choice of adjuvant:**

The comparison of several types of adjuvants showed that phospholipid makes it possible to obtain the best bitterness masking/dissolution compromise (cf. Examples C5 to C11). The preferred phospholipid is soybean lecithin.

**4) Choice of adjuvant content:**

When a phospholipid content of more than 10% is used in the coating formula, a technical difficulty is observed and the method cannot be carried out.

When a phospholipid content of less than 5% is used in the coating formula, it is observed that the method can be carried out, but that a composition agglomeration phenomenon may occur during storage.

Few differences are noted between the percentages of 1, 2 and 3% (cf. Examples C13, C14, C17 and C19).

## 3. Effect of a composition of active ingredient according to the invention on the stability of said active agent with respect to moisture

Nicorandil (N-(2-hydroxyethyl)nicotinamide nitrate) was chosen as an example of an active molecule particularly sensitive to moisture.

It was used according to the method of the invention with, in step E1), granulation using a hot-melt agent, polyethylene glycol 6000 or PEG 6000. This granule obtained is then coated according to step E2) of the method according to the invention at various coating contents with respect to stearic acid: 30% (comparative example), then 54% and 80% (according to the invention). Step E3) is carried out for all the batches.

The resulting coated granules are then placed under stressing temperature and humidity conditions at 40°C/75%RH in a climatic chamber under open conditions (open pillbox).

After 3 days, nicorandil impurity assay analyses are performed by HPLC.

The formulae of the compositions tested, corresponding to the various coating contents, are the following (the percentages are expressed by weight relative to the composition of the coated granule):

|  | Components | Percentage composition (%) |
|---|---|---|
| **COMPOSITION A** | nicorandil | 59.50 |
|  | PEG 6000 | 10.50 |
|  | stearic acid | 30.00 |
|  |  |  |
| **COMPOSITION B** | nicorandil | 38.93 |
|  | PEG 6000 | 6.87 |
|  | stearic acid | 54.20 |
|  |  |  |
| **COMPOSITION C** | nicorandil | 17.00 |
|  | PEG 6000 | 3.00 |
|  | stearic acid | 80.00 |

The results of the nicorandil degradation impurity assay analyses are given in the table below:

|  | COMPOSITION A | COMPOSITION B | COMPOSITION C |
|---|---|---|---|
| **Sum impurities (%)** | 14.61 | 12.63 | 9.14 |
| SG100* (%) | 10.34 | 8.97 | 7.47 |
| Ethyl nicotinamide polymer * (%) | 2.82 | 2.39 | 0.56 |
| * : known major impurities of nicorandil | | | |

These results demonstrate that the coating containing stearic acid significantly improves the stability of nicorandil placed under very high relative humidity conditions. The percentage of major impurities is significantly reduced on compositions B (54% stearic acid) and C (80% stearic acid) compared with composition A (30% stearic acid).

These results show the protective effect, with respect to moisture, of a coating containing stearic acid at greater than 50% in the composition of the coated granule.

### 4. Effect of a composition of active ingredient according to the invention on the inhibition of the anaesthetic effect of said active agent

Drotaverine was chosen as an example of an active molecule having a persistent anaesthetic effect.

It was used according to the method of the invention with, in step E1), a povidone (PVP-K30)-based aqueous-phase granulation.

The resulting granular centre is then coated, in step E2), with stearic acid at various coating contents. Step E3) is finally carried out.

The composition of the coated granule comprises approximately 43% of drotaverine, from 1% to 10% of povidone and various corresponding stearic acid contents.

The introduction of an external phase into the composition of the coated granule thus obtained allows tableting.

The tablets are tasted by volunteers.

The study made it possible to demonstrate that a coating content of less than or equal to 50% was not sufficient to suitably mask the anaesthetic effect of drotaverine. On the other hand, when the coating content is greater than 50%, and preferably about 65% by weight of the composition of the coated granule, the anaesthetic effect of drotaverine is greatly reduced, or even nonexistent.

### 5. Pharmaceutical compositions according to the invention

The percentages indicated below are expressed by total weight of the composition:

### a) Preparation of granules or of a powder to be swallowed, in sachets:

The formulation of the external phase is the following:

- Diluents: from 5% to 90%
- Lubricants: from 0.5% to 2%
- Viscosity modifiers : from 1 % to 10%
- Effervescent agents: from 1 % to 7%
- Sweeteners: from 0.5% to 5%
- Flavours: from 1% to 5%
- Colourants: from 0.1 % to 3%
- Buffering agents: from 0.1 % to 3%
- Sequestering agents: from 0.1 % to 10%.

The external phase is added to the coated granules produced according to the invention in a proportion of from 40% to 70%.

All of the excipients of the external phase, with the exception of the lubricants, are mixed with the coated granules. A lubrication step is then carried out before the placing in sachets.

### b) Preparation of tablets to be chewed, swallowed or sucked:

The formulation of the external phase is the following:

- Diluents: from 5% to 90%
- Lubricants: from 0.5% to 5%
- Sweeteners: from 0.5% to 5%
- Flavours: from 1 % to 5%
- Colourants: from 0.1% to 3%.

The external phase is added to the coated granules produced according to the invention in a proportion of from 40% to 70%.

All of the excipients of the external phase, with the exception of the lubricants, are mixed with the coated granules. A lubrication step is then carried out and the whole is then tableted.

### c) Preparation of orodispersible or dispersible tablets:

The formulation of the external phase is the following:

- Diluents: from 5% to 90%
- Disintegrating agents: from 0 to 30%
- Salivating agents: from 1% to 5%
- Lubricants or flow agents: from 0.5% to 5%
- Sweeteners: from 0.5% to 5%
- Flavours: from 1% to 5%
- Colourants: from 0.1% to 3%.

The external phase is added to the coated granules produced according to the invention in a proportion of from 40% to 70%.
All of the excipients of the external phase, with the exception of the lubricants, are mixed with coated granules. A lubrication step is then carried out and the whole is then tableted.

### d) Orodispersible ketoprofen tablets (25 mg):

Coated granule according to the invention:

- Ketoprofen: 7.81%
- Binder: 0.41%
- Stearic acid: 8.60%

External phase:

- Disintegrating agent: 7%
- Diluents: 73.58%
- Lubricants or flow agents: 1 %
- Flavours and sweeteners: 1.6%.

All of the excipients of the external phase, with the exception of the lubricants, are mixed with the coated granules. A lubrication step is then carried out and the whole is then tableted.

## Claims

1. Method for preparing a composition of medicinal active ingredient comprising (a) a granular centre consisting of grains of active ingredient, agglomerated in the presence of binder, and (b) a layer of coating of said granular centre consisting of fatty matrix, in which composition:

   • the active ingredient represents a minimum of 10%, preferably 20% or else 30% by weight, relative to the weight of the composition of the coated granule; the active ingredient represents a maximum of 48%, preferably a maximum of 40%, the active ingredient preferably represents from 20% to less than 40%, relative to the weight of the composition of the coated granule, more preferentially the active ingredient represents from 30% to 40%, relative to the weight of the composition of the coated granule;
   • the fatty matrix represents more than 50% and up to 85% by weight of the composition of the coated granule, preferably from 51% to 65%, the fatty matrix optionally comprising an adjuvant, preferably chosen from hydrophilic agents, surfactants or mixtures thereof, and the latter representing less than 10%, preferably from 1% to 3% by weight, relative to the weight of the composition of the coated granule;
   • the binder, preferably a hydrophilic agent chosen from hydrophilic polymers or hot-melt agents, represents from 0.2% to 18% by weight, relative to the weight of the composition of the coated granule, preferably the binder represents less than 18% by weight for a hot-melt agent or else preferably the binder represents less than 10% by weight for a hydrophilic polymer;

• the diluent, if necessary, as filler, represents a content of 0 to 39%, relative to the weight of the composition of the coated granule;
• the lubricant, if necessary, as flow agent, represents a content of 0 to 1.8%, relative to the weight of the composition of the coated granule;

the method comprising the steps of:

E1) preparing the granular centre by spraying an aqueous solution comprising the binder or by spraying the binder in the molten state onto the active ingredient alone or as a mixture with a diluent and/or a lubricant;
E2) coating by spraying, onto the granules, said fatty matrix pre-melted in a melting kettle at a temperature approximately 10 to 20°C above its melting point;
E3) cooling the composition obtained.

2. Method for preparing a composition according to Claim 2, in which:

• the active ingredient represents a minimum of 10% and a maximum of 48%, preferably from 20% to less than 40% by weight of the composition of the coated granule, and
• the fatty matrix comprising an adjuvant represents more than 50% by weight and up to 85% by weight of the composition of the coated granule.

3. Method for preparing a composition according to Claim 2, in which:

• the active ingredient represents from 30% to 40%, relative to the weight of the composition of the coated granule, and
• the fatty matrix represents from 51% up to 65% by weight of the composition of the coated granule.

4. Method according to one of Claims 1 to 3, in which the size of the coated granules obtained at the end of step E3) is less than 500 $\mu$m, preferably less than 355 $\mu$m, preferably ranging from 100 to 300 $\mu$m.

5. Method according to one of Claims 1 to 4, in which the particle size of the final product obtained at the end of step E3) is distributed according to the following range:

• less than 15% by weight of the coated granules are greater than 500 $\mu$m;
• more than 80% by weight, preferably more than 90% by weight, of the coated granules are between 355 and 90 $\mu$m; and
• less than 20% by weight, preferably less than 5% by weight, of the coated granules are less than 90 $\mu$m.

6. Method according to one of Claims 1 to 5, in which the aqueous solution used in step E1 comprises, as binder, a hydrophilic polymer preferably chosen from the group of cellulosic derivatives (hydroxypropylcellulose), povidone (polyvinylpyrrolidone), sucrose, gums, starches, gelatin and macrogols (polyethylene glycols), which represents approximately from 15% to 45%, preferably from 20% to 40% by weight of said aqueous solution.

7. Method according to one of Claims 1 to 6, in which the binder used in step E1 for the granulation is a hot-melt agent chosen from macrogols (PEGs), sucroesters or else poloxamers, and represents approximately from 0.2% to 20%, preferably from 1% to 15% by weight, relative to the amount of active ingredient to be granulated in step E1).

8. Method according to one of Claims 1 to 7, in which the fatty matrix consists of saturated fatty acids with long C14 to C22, preferably C16 to C18, carbon-based chains, pure or as mixtures, and/or their corresponding fatty alcohols.

9. Method according to one of Claims 1 to 8, in which the fatty matrix consists of stearic acid, palmitic acid, myristic acid, pure or as mixtures, and/or their corresponding fatty alcohols.

10. Method according to Claim 9, in which the fatty matrix consists of stearic acid.

11. Method according to one of Claims 1 to 10, in which the fatty matrix comprises an adjuvant chosen from the group of surfactants (phospholipid, polysorbate, lauryl sulphate), hydrophilic excipients such as sucrose, polyols, cellulose, lactose, silica, dicalcium phosphate, carbonates, starch, macrogols and agents which are soluble at acidic pH (methacrylic derivatives), pure or as mixtures.

**12.** Method according to Claim 11, in which the adjuvant as a mixture with the fatty matrix is soybean lecithin.

**13.** Method according to one of Claims 1 to 8, in which said fatty matrix coating the granular centre consists of stearic acid and in which said fatty matrix comprises soybean lecithin as adjuvant.

**14.** Method according to one of Claims 1 to 13, in which the percentage by weight of the adjuvant added to the fat in step E2 is less than 10% by weight, preferably less than 5% by weight, preferably ranging from 1% to 3% by weight, relative to the weight of the final composition.

**15.** Method according to one of Claims 1 to 14, in which the percentage by weight of the binder constituting the coating of the granule obtained in step E1 represents from 1% to 5% by weight, relative to the weight of the final composition, for a hydrophilic polymer and from 0.2% to 18% for a hot-melt agent.

**16.** Method according to one of Claims 1 to 15, in which the active ingredient is chosen from the group consisting of antibiotics such as cephalosporins and macrolides, advantageously chosen from: pristinamycin, cefpodoxime, roxithromycin, spiramycin, rovamycin and levofloxacin, or the group consisting of corticoids such as prednisolone or methylprednisolone, or else the group consisting of NSAIDs such as tiaprofenic acid, ketoprofen, ketoprofen lysinate or ibuprofen, or the group consisting of analgesics such as paracetamol, or else the group consisting of zopiclone, riluzole, zolpidem, clobazam, thiocolchicoside, drotaverine hydrochloride or base, amodiaquine hydrochloride, diltiazem, levocetirizin, mizolastine, dronedarone, celivarone, dramamine, ramipril, irbesartan, clopidogrel, leflunomide, nicorandil, lysine acetyl salicylate, magnesium citrate, levothyroxine, sodium valproate, rifampicin, artesunate and omeprazole.

**17.** Method according to one of Claims 1 to 16, followed by a step E4 of formulating the coated granules obtained in step E3 with excipients such as diluents, fillers, viscosity modifiers, disintegrating agents, effervescent agents, colourants, sweeteners, salivating agents, flavourings, buffers and sequestering agents, flow agents and lubricants for the manufacture of an oral formulation in the form of granules for sachets, granules for oral suspension, or granules for conventional tablets or for orodispersible tablets.

**18.** Composition of medicinal active ingredient which can be obtained according to the method described in one of Claims 1 to 17.

**19.** Pharmaceutical composition comprising the composition of medicinal active ingredient as defined in Claim 18 or prepared according to the method of any one of Claims 1 to 17.

**20.** Composition according to Claim 19, **characterized in that** it is in the form of sachets for oral suspension; tablets to be chewed, swallowed or sucked; or orodispersible or water-dispersible tabets; or powder to be swallowed.

**21.** Use of a composition of medicinal active ingredient as defined in Claim 18 or prepared according to the method of any one of Claims 1 to 17, for preparing a pharmaceutical composition in the form of sachets for oral suspension, powders to be swallowed, tablets to be chewed, tablets to be swallowed, tablets to be sucked, orodispersible tablets or water-dispersible tablets, and having a masked taste.


**Patentansprüche**

**1.** Verfahren zur Herstellung einer Zusammensetzung eines medizinischen Wirkstoffs, umfassend (a) ein granuläres Zentrum, das aus in Gegenwart von Bindemittel agglomerierten Wirkstoffkörnern besteht, und (b) eine Beschichtungsschicht für das granuläre Zentrum, die aus Fettmatrix besteht, wobei in der Zusammensetzung:

• der Wirkstoff mindestens 10 Gew.-%, vorzugsweise 20 Gew.-% oder auch 30 Gew.-%, bezogen auf das Gewicht der Zusammensetzung des beschichteten Granulats, ausmacht; der Wirkstoff höchstens 48% und vorzugsweise höchstens 40% ausmacht, der Wirkstoff vorzugsweise 20% bis weniger als 40%, bezogen auf das Gewicht der Zusammensetzung des beschichteten Granulats ausmacht, der Wirkstoff weiter bevorzugt 30% bis 40%, bezogen auf das Gewicht der Zusammensetzung des beschichteten Granulats, ausmacht;
• die Fettmatrix mehr als 50 Gew.-% und bis zu 85 Gew.-% der Zusammensetzung des beschichteten Granulats, vorzugsweise 51% bis 65%, ausmacht, die Fettmatrix gegebenenfalls einen Hilfsstoff umfasst, der vorzugsweise aus hydrophilen Mitteln, Tensiden oder Mischungen davon ausgewählt ist, wobei letztere weniger als 10 Gew.-

% und vorzugsweise 1 bis 3 Gew.-%, bezogen auf das Gewicht der Zusammensetzung des beschichteten Granulats, ausmachen;

• das Bindemittel, vorzugsweise ein hydrophiles Mittel, das aus hydrophilen Polymeren oder wärmeschmelzbaren Mitteln ausgewählt ist, 0,2 bis 18 Gew.-%, bezogen auf das Gewicht der Zusammensetzung des beschichteten Granulats, ausmacht, das Bindemittel vorzugsweise weniger als 18 Gew.-% für ein wärmeschmelzbares Mittel ausmacht oder auch das Bindemittel vorzugsweise weniger als 10 Gew.-% für ein hydrophiles Polymer ausmacht;

• das Verdünnungsmittel, sofern notwendig, als Füllstoff einen Gehalt von 0 bis 39%, bezogen auf das Gewicht der Zusammensetzung des beschichteten Granulats, ausmacht;

• das Gleitmittel, sofern notwendig, als Fließmittel einen Gehalt von 0 bis 1,8%, bezogen auf das Gewicht der Zusammensetzung des beschichteten Granulats, ausmacht;

wobei das Verfahren die folgenden Schritte umfasst:

E1) Herstellen des granulären Zentrums durch Aufsprühen einer wässrigen Lösung, die das Bindemittel umfasst, oder durch Aufsprühen des Bindemittels in schmelzflüssigem Zustand auf den Wirkstoff alleine oder als Mischung mit einem Verdünnungsmittel und/oder Gleitmittel;

E2) Beschichten durch Aufsprühen der Fettmatrix, die in einem Schmelzkessel bei einer ungefähr 10 bis 20°C über ihrem Schmelzpunkt liegenden Temperatur vorher aufgeschmolzen wurde, auf das Granulat;

E3) Abkühlen der erhaltenen Zusammensetzung.

2. Verfahren zur Herstellung einer Zusammensetzung nach Anspruch 2, bei dem:

• der Wirkstoff mindestens 10 Gew.-% und maximal 48 Gew.-% und vorzugsweise 20 Gew.-% bis weniger als 40 Gew.-% der Zusammensetzung des beschichteten Granulats ausmacht und

• die Fettmatrix, die einen Hilfsstoff umfasst, mehr als 50 Gew.-% und bis zu 85 Gew.-% der Zusammensetzung des beschichteten Granulats ausmacht.

3. Verfahren zur Herstellung einer Zusammensetzung nach Anspruch 2, bei dem:

• der Wirkstoff mindestens 30% bis 40%, bezogen auf das Gewicht der Zusammensetzung des beschichteten Granulats, ausmacht und

• die Fettmatrix 51 bis 65 Gew.-% der Zusammensetzung des beschichteten Granulats ausmacht.

4. Verfahren nach einem der Ansprüche 1 bis 3, bei dem die Größe des am Ende von Schritt E3) erhaltenen beschichteten Granulats weniger als 500 μm und vorzugsweise weniger als 355 μm beträgt und vorzugsweise im Bereich von 100 bis 300 μm liegt.

5. Verfahren nach einem der Ansprüche 1 bis 4, bei dem die Teilchengröße des am Ende von Schritt E3) erhaltenen Endprodukts gemäß dem folgenden Bereich verteilt ist:

• weniger als 15 Gew.-% des beschichteten Granulats sind größer als 500 μm;

• mehr als 80 Gew.-% und vorzugsweise mehr als 90 Gew.-% des beschichteten Granulats sind zwischen 355 und 90 μm; und

• weniger als 20 Gew.-% und vorzugsweise weniger als 5 Gew.-% des beschichteten Granulats sind kleiner als 90 μm.

6. Verfahren nach einem der Ansprüche 1 bis 5, bei dem die in Schritt E1 verwendete wässrige Lösung als Bindemittel ein hydrophiles Polymer, vorzugsweise ausgewählt aus der Gruppe Cellulosederivate (Hydroxypropylcellulose), Povidon (Polyvinylpyrrolidon), Saccharose, Gummen, Stärken, Gelatine und Macrogole (Polyethylenglykole), umfasst, das ungefähr 15 bis 45 Gew.-% und vorzugsweise 20 bis 40 Gew.-% der wässrigen Lösung ausmacht.

7. Verfahren nach einem der Ansprüche 1 bis 6, bei dem das in Schritt E1 für die Granulierung verwendete Bindemittel ein wärmeschmelzbares Mittel, ausgewählt aus Macrogolen (PEGs), Zuckerestern oder auch Poloxameren, ist und ungefähr 0,2 bis 20 Gew.-% und vorzugsweise 1 bis 15 Gew.-%, bezogen auf die Menge von in Schritt E1) zu granulierendem Wirkstoff, ausmacht.

8. Verfahren nach einem der Ansprüche 1 bis 7, bei dem die Fettmatrix aus gesättigten Fettsäuren mit langen C14- bis C22- und vorzugsweise C16- bis C18-Ketten auf Kohlenstoffbasis, rein oder als Mischungen, und/oder den entsprechenden Fettalkoholen besteht.

9. Verfahren nach einem der Ansprüche 1 bis 8, bei dem die Fettmatrix aus Stearinsäure, Palmitinsäure, Myristinsäure, rein oder als Mischungen, und/oder den entsprechenden Fettalkoholen besteht.

10. Verfahren nach Anspruch 9, bei dem die Fettmatrix aus Stearinsäure besteht.

11. Verfahren nach einem der Ansprüche 1 bis 10, bei dem die Fettmatrix einen Hilfsstoff, ausgewählt aus der Gruppe Tenside (Phospholipid, Polysorbat, Laurylsulfat), hydrophile Exzipienten wie Saccharose, Polyole, Cellulose, Lactose, Siliciumdioxid, Dicalciumphosphat, Carbonate, Stärke, Macrogole und Mittel, die bei saurem pH-Wert löslich sind (Methacrylderivate), rein oder als Mischungen, umfasst.

12. Verfahren nach Anspruch 11, bei dem es sich bei dem Hilfsstoff als Mischung mit der Fettmatrix um Sojabohnenlecithin handelt.

13. Verfahren nach einem der Ansprüche 1 bis 8, bei dem die das granuläre Zentrum beschichtende Fettmatrix aus Stearinsäure besteht und die Fettmatrix Sojabohnenlecithin als Hilfsstoff umfasst.

14. Verfahren nach einem der Ansprüche 1 bis 13, bei dem der Gewichtsprozentanteil des dem Fett in Schritt E2 zugesetzten Hilfsstoffs weniger als 10 Gew.-% und vorzugsweise weniger als 5 Gew.-% beträgt und vorzugsweise im Bereich von 1 bis 3 Gew.-% liegt, bezogen auf das Gewicht der Endzusammensetzung.

15. Verfahren nach einem der Ansprüche 1 bis 14, bei dem der Gewichtsprozentanteil des Bindemittels, das die Beschichtung des in Schritt E1 erhaltenen Granulats bildet, 1 bis 5 Gew.-%, bezogen auf das Gewicht der Endzusammensetzung, für ein hydrophiles Polymer und 0,2% bis 18% für ein wärmeschmelzbares Mittel beträgt.

16. Verfahren nach einem der Ansprüche 1 bis 15, bei dem man den Wirkstoff aus der Gruppe bestehend aus Antibiotika wie Cephalosporinen und Macroliden, vorteilhafterweise ausgewählt aus: Pristinamycin, Cefpodoxim, Roxithromycin, Spiramycin, Rovamycin und Levofloxacin, oder der Gruppe bestehend aus Corticoiden wie Prednisolon oder Methylprednisolon oder auch der Gruppe bestehend aus NSAIDs wie Tiaprofensäure, Ketoprofen, Ketoprofenlysinat oder Ibuprofen oder der Gruppe bestehend aus Analgetika wie Paracetamol oder auch der Gruppe bestehend aus Zopiclon, Riluzol, Zolpidem, Clobazam, Thiocolchicosid, Drotaverin-hydrochlorid oder -Base, Amodiaquin-hydrochlorid, Diltiazem, Levocetirizin,
    Mizolastin, Dronedaron, Celivaron, Dramamin, Ramipril, Irbesartan, Clopidogrel, Leflunomid, Nicorandil, Lysinacetylsalicylat, Magnesiumcitrat, Levothyroxin, Natriumvalproat, Rifampicin, Artesunat und Omeprazol auswählt.

17. Verfahren nach einem der Ansprüche 1 bis 16, gefolgt von einem Schritt E4 des Formulierens des in Schritt E3 erhaltenen beschichteten Granulats mit Exzipienten wie Verdünnungsmitteln, Füllstoffen, Viskositätsmodifikatoren, Zerfallsmitteln, Brausemitteln, Farbmitteln, Süßungsmitteln, Speichelmitteln, Geschmacksstoffen, Puffersubstanzen und Sequestriermitteln, Fließmitteln und Gleitmitteln zur Herstellung einer oralen Formulierung in Form von Granulaten für Beutel, Granulaten für eine orale Suspension oder Granulaten für herkömmliche Tabletten oder orodispersible Tabletten.

18. Zusammensetzung von medizinischem Wirkstoff, die nach dem in einem der Ansprüche 1 bis 17 beschriebenen Verfahren erhältlich ist.

19. Pharmazeutische Zusammensetzung, umfassend die wie in Anspruch 18 definierte oder gemäß dem Verfahren nach einem der Ansprüche 1 bis 17 hergestellte Zusammensetzung von medizinischem Wirkstoff.

20. Zusammensetzung nach Anspruch 19, **dadurch gekennzeichnet, dass** sie in Form von Beuteln für eine orale Suspension, Kau-, Schluck- oder Lutschtabletten oder orodispersiblen oder wasserdispergierbaren Tabletten oder Pulvern zum Schlucken vorliegt.

21. Verwendung einer in Anspruch 18 definierten oder gemäß dem Verfahren nach einem der Ansprüche 1 bis 17 hergestellten Zusammensetzung von medizinischem Wirkstoff zur Herstellung einer pharmazeutischen Zusammensetzung in Form von Beuteln für eine orale Suspension, Kau-, Schluck- oder Lutschtabletten oder orodispersiblen oder wasserdispergierbaren Tabletten oder Pulvern zum Schlucken und mit einem maskierten Geschmack.

**Revendications**

1. Procédé pour préparer une composition de substance active médicinale comprenant (a) un centre granulaire constitué de grains de substance active, agglomérés en présence d'un liant, et (b) une couche d'enrobage dudit centre granulaire constituée de matrice grasse, composition dans laquelle :

    • la substance active représente un minimum de 10 %, de préférence 20 % ou sinon 30 % en poids, par rapport au poids de la composition du granule enrobé ; la substance active représente un maximum de 48 %, de préférence un maximum de 40 %, la substance active représente de préférence de 20 % à moins de 40 %, par rapport au poids de la composition du granule enrobé, de façon plus préférable la substance active représente de 30 % à 40 %, par rapport au poids de la composition du granule enrobé ;
    • la matrice grasse représente plus de 50 % et jusqu'à 85 % en poids de la composition du granule enrobé, de préférence de 51 % à 65 %, de la matrice grasse comprenant facultativement un adjuvant, de préférence choisi parmi des agents hydrophiles, des tensioactifs ou des mélanges de ceux-ci, et ce dernier représentant moins de 10 %, de préférence de 1 % à 3 % en poids, par rapport au poids de la composition du granule enrobé ;
    • le liant, de préférence un agent hydrophile choisi parmi des polymères hydrophiles ou des agents thermofusibles, représente de 0,2 % à 18 % en poids, par rapport au poids de la composition du granule enrobé, de préférence le liant représente moins de 18 % en poids pour un agent thermofusible ou sinon, de préférence, le liant représente moins de 10 % en poids pour un polymère hydrophile ;
    • le diluant, si nécessaire, en tant que charge, représente une teneur de 0 à 39 %, par rapport au poids de la composition du granule enrobé ;
    • le lubrifiant, si nécessaire, en tant qu'agent de fluidité, représente une teneur de 0 à 1,8 %, par rapport au poids de la composition du granule enrobé ;

    le procédé comprenant les étapes consistant à :

    E1) préparer le centre granulaire par pulvérisation d'une solution aqueuse comprenant le liant ou par pulvérisation du liant à l'état fondu sur la substance active seule ou sous forme de mélange avec un diluant et/ou un lubrifiant ;
    E2) enrober par pulvérisation, sur les granules,
    ladite matrice grasse préfondue dans une chaudière de fonte à une température d'approximativement 10 à 20 °C au-dessus de son point de fusion ;
    E3) refroidir la composition obtenue.

2. Procédé pour préparer une composition selon la revendication 2, dans lequel :

    • la substance active représente un minimum de 10 % et un maximum de 48 %, de préférence de 20 % à moins de 40 % en poids de la composition du granule enrobé, et
    • la matrice grasse comprenant un adjuvant représente plus de 50 % et jusqu'à 85 % en poids de la composition du granule enrobé.

3. Procédé pour préparer une composition selon la revendication 2, dans lequel :

    • la substance active représente de 30 % à 40 %, par rapport au poids de la composition du granule enrobé, et
    • la matrice grasse représente de 51 % jusqu'à 65 % en poids de la composition du granule enrobé.

4. Procédé selon une des revendications 1 à 3, dans lequel la taille des granules enrobés obtenus à la fin de l'étape E3) est inférieure à 500 $\mu$m, de préférence inférieure à 355 $\mu$m, de préférence dans la plage de 100 à 300 $\mu$m.

5. Procédé selon une des revendications 1 à 4, dans lequel la taille de particule du produit final obtenu à la fin de l'étape E3) est distribuée dans la plage suivante :

    • moins de 15 % en poids des granules enrobés sont supérieurs à 500 $\mu$m ;
    • plus de 80 % en poids, de préférence plus de 90 % en poids, des granules enrobés sont compris entre 355 et 90 $\mu$m ; et
    • moins de 20 % en poids, de préférence moins de 5 % en poids, des granules enrobés sont inférieurs à 90 $\mu$m.

6. Procédé selon une des revendications 1 à 5, dans lequel la solution aqueuse utilisée dans l'étape E1 comprend, en tant que liant, un polymère hydrophile de préférence choisi dans le groupe de dérivés cellulosiques (hydroxypropylcellulose), povidone (polyvinylpyrrolidone), saccharose, gommes, amidons, gélatine et macrogols (polyéthylèneglycols), qui représente approximativement de 15 % à 45 %, de préférence 20 % à 40 % en poids de ladite solution aqueuse.

**7.** Procédé selon une des revendications 1 à 6, dans lequel le liant utilisé dans l'étape E1 pour la granulation est un agent thermofusible choisi parmi des macrogols (PEG), des esters de sucre ou sinon des poloxamers, et représente approximativement de 0,2 % à 20 %, de préférence de 1 % à 15 % en poids, par rapport à la quantité de substance active à granuler dans l'étape E1).

**8.** Procédé selon une des revendications 1 à 7, dans lequel la matrice grasse est constituée d'acides gras saturés avec des chaînes à base de carbone longues de C14 à C22, de préférence de C16 à C18, purs ou en mélanges, et/ou leurs alcools gras correspondants.

**9.** Procédé selon une des revendications 1 à 8, dans lequel la matrice grasse est constituée d'acide stéarique, d'acide palmitique, d'acide myristique, purs ou en mélanges, et/ou leurs alcools gras correspondants.

**10.** Procédé selon la revendication 9, dans lequel la matrice grasse est constituée d'acide stéarique.

**11.** Procédé selon une des revendications 1 à 10, dans lequel la matrice grasse comprend un adjuvant choisi dans le groupe de tensioactifs (phospholipide, polysorbate, laurylsulfate), excipients hydrophiles tels que le saccharose, des polyols, la cellulose, le lactose, la silice, le phosphate dicalcique, les carbonates, l'amidon, les macrogols et des agents qui sont solubles à pH acide (dérivés méthacryliques), purs ou en mélanges.

**12.** Procédé selon la revendication 11, dans lequel l'adjuvant en mélange avec la matrice grasse est la lécithine de soja.

**13.** Procédé selon une des revendications 1 à 8, dans lequel ladite matrice grasse enrobant le centre granulaire est constituée d'acide stéarique et dans lequel ladite matrice grasse comprend de la lécithine de soja en tant qu'adjuvant.

**14.** Procédé selon une des revendications 1 à 13, dans lequel le pourcentage en poids de l'adjuvant ajouté à la graisse dans l'étape E2 est inférieur à 10 % en poids, de préférence inférieur à 5 % en poids, de préférence dans la plage de 1 % à 3 % en poids, par rapport au poids de la composition finale.

**15.** Procédé selon une des revendications 1 à 14, dans lequel le pourcentage en poids du liant constituant l'enrobage du granule obtenu dans l'étape E1 représente de 1 % à 5 % en poids, par rapport au poids de la composition finale, pour un polymère hydrophile et de 0,2 % à 18 % pour un agent thermofusible.

**16.** Procédé selon une des revendications 1 à 15, dans lequel la substance active est choisie dans le groupe constitué d'antibiotiques tels que des céphalosporines et des macrolides, avantageusement choisis parmi : la pristinamycine, le cefpodoxime, la roxithromycine, la spiramycine, la rovamycine et la lévofloxacine, ou le groupe constitué de corticoïdes tels que la prednisolone ou la méthylprednisolone, ou sinon le groupe constitué de NSAID tels que l'acide tiaprofénique, le kétoprofène, le kétoprofène lysinate ou l'ibuprofène, ou le groupe constitué d'analgésiques tels que le paracétamol, ou sinon le groupe constitué de la zopiclone, le riluzole, le zolpidem, le clobazam, le thiocolchicoside, le drotavérine chlorhydrate ou base, l'amodiaquine chlorhydrate, le diltiazem, la lévocétirizine, la mizolastine, la dronédarone, la célivarone, la dramamine, le ramipril, l'irbésartan, le clopidogrel, le léflunomide, le nicorandil, l'acétylsalicylate de lysine, le citrate de magnésium, la lévothyroxine, le valproate de sodium, la rifampicine, l'artésunate et l'oméprazole.

**17.** Procédé selon une des revendications 1 à 16, suivi par une étape E4 de formulation des granules enrobés obtenus dans l'étape E3 avec des excipients tels que des diluants, des charges, des modifieurs de viscosité, des agents délitants, des agents effervescents, des colorants, des édulcorants, des agents de salivation, des arômes, des tampons et des agents séquestrants, des agents d'écoulement et des lubrifiants pour la fabrication d'une formulation orale sous la forme de granules pour sachets, granules pour suspension orale, ou granules pour des comprimés conventionnels ou pour des comprimés orodispersibles.

**18.** Composition de substance active médicinale qui peut être obtenue selon le procédé décrit dans une des revendications 1 à 17.

**19.** Composition pharmaceutique comprenant la composition de substance active médicinale telle que définie dans la revendication 18 ou préparée selon le procédé de l'une quelconque des revendications 1 à 17.

**20.** Composition selon la revendication 19, **caractérisée en ce qu'**elle est sous la forme de sachets pour suspension orale ; de comprimés à mâcher, à avaler ou à sucer ; ou de comprimés orodispersibles ou dispersibles dans l'eau ; ou de poudre à avaler.

**21.** Utilisation d'une composition de substance active médicinale telle que définie dans la revendication 18 ou préparée selon le procédé de l'une quelconque des revendications 1 à 17, pour préparer une composition pharmaceutique sous la forme de sachets pour suspension orale, de poudres à avaler, de comprimés à mâcher, de comprimés à avaler, de comprimés à sucer, de comprimés orodispersibles ou de comprimés dispersibles dans l'eau, et ayant un goût masqué.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004058137 A
- WO 0207706 A
- WO 9718798 A